# EUROPÄISCHE PATENTSCHRIFT

(11) **EP 1 159 000 B1**
(45) Veröffentlichungstag und Bekanntmachung des Hinweises auf die Patenterteilung: **25.09.2002**
(21) Anmeldenummer: 00914103.7
(22) Anmeldetag: 22.02.2000
(51) Int. Cl.: A61K 38/52, A61K 39/395, A61K 48/00, A61P 17/00, A61K 38/51

(54) **MITTEL ZUR BEHANDLUNG VON PIGMENTIERUNGSSTÖRUNGEN**
PREPARATION FOR THE TREATMENT OF PIGMENTATION DISORDERS
AGENT POUR TRAITER DES TROUBLES DE LA PIGMENTATION

(30) Priorität: 25.02.1999 DE 19908242; 27.07.1999 DE 19934458
(43) Veröffentlichungstag der Anmeldung: 05.12.2001
(73) Patentinhaber: Pogge von Strandmann, Elke, 44807 Bochum (DE)
(72) Erfinder: Pogge von Strandmann, Elke, 44807 Bochum (DE)
(74) Vertreter: Thiel, Christian
(86) Internationale Anmeldenummer: EP0001822
(87) Internationale Veröffentlichungsnummer: WO00050070

(56) Entgegenhaltungen:
- SCHALLREUTER KARIN U ET AL: "Defective tetrahydrobiopterin and catecholamine biosynthesis in the depigmentation disorder vitiligo." BIOCHIMICA ET BIOPHYSICA ACTA, Bd. 1226, Nr. 2, 1994, Seiten 181-192, XP000946158 ISSN: 0006-3002
- STRANDMANN ELKE POGGE V ET AL: "The bifunctional protein DCoH/PCD, a transcription factor with a cytoplasmic enzymatic activity, is a maternal factor in the rat egg and expressed tissue specifically during embryogenesis." INTERNATIONAL JOURNAL OF DEVELOPMENTAL BIOLOGY, Bd. 42, Nr. 1, Januar 1998 (1998-01), Seiten 53-59, XP000946178 ISSN: 0214-6282
- LI LINGNA ET AL: "Topical liposome delivery of molecules to hair follicles in mice." JOURNAL OF DERMATOLOGICAL SCIENCE, Bd. 14, Nr. 2, 1997, Seiten 101-108, XP000946174 ISSN: 0923-1811
- V STRANDMANN ELKE POGGE ET AL: "Ectopic pigmentation in Xenopus in response to DCoH/PCD, the cofactor of HNF1 transcription factor/pterin-4alpha-carbinolamine dehydratase." MECHANISMS OF DEVELOPMENT, Bd. 91, Nr. 1-2, 1. März 2000 (2000-03-01), Seiten 53-60, XP000946160 ISSN: 0925-4773

## Beschreibung

Die Erfindung betrifft ein Mittel zur Behandlung von Pigmentierungsstörungen des menschlichen und tierischen Körpers unter Verwendung des Proteins DCoH, der dafür codierenden DNA und/oder RNA als Wirkstoff.

Das Enzym DCoH ist der Dimerisierungscofaktor der HNF-1 (Hepatocyte Nuclear Factor-1) homeodomänen Proteine (HNF-1α und HNF-1β). Durch diesen Mechanismus ist DCoH an der Kontrolle der Genexpression beteiligt, siehe D.B. Mendel et al., Science 254 (1991, 1762). Gleichzeitig ist das Protein als Pterin-4α-carbinolamindehydratase PCD bekannt und an der Tetrahydrobiopterinregenerierung beteiligt, siehe B.A. Citron et al., Proc. Natl. Acad. Sci. USA 89, 1992, 11891. PCD greift dabei in die Biosynthese von L-Tyrosin aus L-Phenylalanin ein. In letzterem Cyclus ist PCD zusammen mit Phenylalaninhydroxylase eingebunden und an der Regenerierung von (6R)5,6,7,8-Tetrahydrobiopterin beteiligt. Unabhängig davon wird DCoH/PCD aber auch im vertebraten Ei, im pigmentierten Epithel des Auges, in der Haut und im Gehirn gefunden, siehe E. Pogge v. Strandmann und G.U. Ryffel, Development 121 (1995), 1217; E. Pogge v. Strandmann et al. Int. J. Dev. Biol. 42 (1998), 53.

Nach ersten Daten über die Reinigung und Klonierung von DCoH von D.B. Mendel et al, Science 254 (1991), 1762 wurde so wohl die Peptidsequenz als auch die dafür codierende Nukleinsäuresequenz von Human-, Maus- und Ratten-DCoH in US-A 5 403 712 und 5 620 887 erstmalig beschrieben. Bezüglich aller relevanter Strukturdaten wird auf diese Veröffentlichungen verwiesen. DCoH/PCD hat sich als mehr oder weniger universelles Prinzip in der Entwicklung von Wirbeltieren, insbesondere auch für die Frühentwicklung erwiesen. Es weist sowohl katalytische wie regulierende Eigenschaften auf und ist in einer größeren Anzahl von Zelltypen präsent. In einigen dieser Zelltypen ist es mit den nuklearen Transkriptionsfaktoren HNF-1α und HNF-1β verschwistert, in anderen in den Phenylalaninhydroxylase-Enzymkomplex eingebunden. Das Vorkommen von DCoH/PCD in Zelltypen in Abwesenheit von HNF-1 oder Phenylalaninhydroxylase weist darauf hin, daß das Protein auch mit davon verschiedenen zellularen Partnern zusammenwirkt.

Eine bekannte Pigmentierungsstörung ist beispielsweise Vitiligo. Vitiligo zeichnet sich durch weiße, pigmentfreie, meist langsam größer werdende Flecke mit hyperpigmentiertem Rand aus und wird auf eine Hemmung der Melaninsynthese zurückgeführt. Die Erscheinung kann in Verbindung mit anderen Erkrankungen, wie Diabetes mellitus, auftreten. Eine ursächliche Behandlung ist bislang nicht möglich. Vitiligo scheint mit einem DcoH-Mangel einherzugehen.

Andere Depigmentierungsstörungen sind beispielsweise Formen von Canities, also Grauhaarigkeit, sowohl in der vorzeitigen wie der im höheren Alter auftretenden physiologischen Form. Auch hier sind ursächliche Therapien nicht bekannt.

Eine Erkrankung, die mit Depigmentierungserscheinungen verbunden ist, ist Alopecia areata, ein lokaler, plötzlich einsetzender und ursächlich unklarer Haarausfall, insbesondere auch der Kopfhaare. Bei diesem Haarausfall bleiben die Haarfollikel erhalten; es kommt zu spontanen Remissionen, bei denen die betroffenen Haare (zunächst) weiß nachwachsen. Die Erkrankung ist also mit einer Pigmentierungsstörung verbunden.

Ferner sind das Usher- und das Waardenburg-Syndrom mit Pigmentierungsstörungen verbunden. Darüber hinaus sind zahlreiche Störungen bekannt, die mit einer (lokalen) Überpigmentierung verbunden sind.

Bei den vorstehend genannten Erscheinungen und Erkrankungen sind die Wirkungen eingehend beschrieben, ohne daß aber die Ursachen geklärt werden konnten. Die Vermutung, daß das Peptid DCoH/PCD dabei eine Rolle spielen könnte, wurde in Bezug auf Vitiligo geäußert, jedoch nicht bewiesen. Tatsächlich deutet vieles darauf hin, daß die beobachtete Verminderung der enzymatischen Aktivität von DCoH bei milden Formen von Hyperphenylalaninämie und Vitiligo eine Folge und nicht die Ursache für diese Störungen ist. Nachgewiesen wurde in den betroffenen Zellen ein Überangebot des für die Bildung von L-Tyrosin aus L-Phenylalanin notwendigen Cofaktors Tetrahydrobiopterin.

Insgesamt besteht ein Bedarf an Mitteln, die geeignet sind, Pigmentierungsstörungen des menschlichen und tierischen Körpers und damit verwandter Erscheinungen zu behandeln.

Es wurde jetzt überraschend gefunden, daß DCoH/PCD geeignet ist, de novo pigmentierte Zellen zu schaffen. Dabei ist DCoH nicht Glied in der Kette der Melaninsynthese, dessen Ausfall zur Depigmentierung führt, sondern es findet eine "künstliche Überexpression" statt, die die Pigmentierung auslöst. Es wurde ferner gefunden, daß DcoH-Antiserum geeignet ist, eine Pigmentierung abzuschwächen oder (deren Entstehung) zu verhindern.

Gegenstand der Erfindung ist somit ein Mittel zur Behandlung von Pigmentierungsstörungen, bei dem eine physiologisch wirksame Menge des Proteins DCoH, der dafür codierenden DNA und/oder RNA oder von DcoH-Antikörpern als Wirkstoff in einem pharmazeutisch annehmbaren Trägermaterial enthalten ist.

Das erfindungsgemäße Mittel enthält das die Pigmentierung auslösende Protein DCoH oder, alternativ oder ergänzend, die dafür codierende DNA und/oder RNA oder alternativ, DcoH-Antikörper bzw. -Antiserum als Wirkstoff.

Experimentell wurde der Effekt von DCoH/PCD nach der Injektion von mRNA in Oocyten gezeigt. Da die RNA in der Zelle das Produkt der Transkription der entsprechenden DNA ist, und selbst in das Protein übersetzt wird, ist die Wirksamkeit aller drei Wirkstoffe, Protein, DNA und RNA, sichergestellt, wobei gewisse Spektren bei der Stärke und Dauer der Wirkung auftreten können. Protein- und Nuklein-Säure-Sequenz von DCoH und der dafür codierenden DNA sind in US-A 5 403 712 und 5 620 887 beschrieben. Die Herstellung von Antiserum ist in Development 121, 1217 (1995) beschrieben. Im einzelnen kommen sowohl poly- wie auch monoklonale Antikörper für die erfindungsgemäßen Zwecke in Frage.

Das erfindungsgemäße Mittel zur Behandlung von Pigmentierungsstörungen enthält den Wirkstoff in einem üblichen pharmazeutisch annehmbaren und verträglichen Trägermaterial. Solche Trägermaterialien sind sowohl für die topische als auch die parenterale Verabreichung bekannt. Möglich sind ferner gentherapeutische Verabreichungsformen, bei denen das für DCoH decodierende Gen in die Zielzelle eingeführt wird. Die Wirkstoffkonzentrationen sind auf dem Gebiet der Protein/DNA/RNA-Therapie üblich und orientieren sich an den in den Zielzellen normalen Konzentrationen, d. h. liegen auf normaler Höhe oder darüber.

Bei der topischen Verabreichung kann das Mittel auf die Haut aufgebracht werden, wobei der Wirkstoff in einem transdermal wirksamen Trägermaterial enthalten ist. Solche Trägermaterialien, die hier als Penetrationshilfe oder Penetrationsbeschleuniger ausgebildet sind und meist in Form von Liposomen vorliegen, sind bekannt. Des weiteren ist das Aufbringen mittels eines transdermalen Systems möglich und insbesondere bei der Behandlung lokaler Störungen sinnvoll.

Bei der topischen Anwendung, die sich auf bestimmte Regionen oder Zellen richtet, beispielsweise bei überpigmentierten oder depigmentierten Hautpartien oder Bereichen der Behaarung, empfiehlt sich insbesondere die Verwendung von hierfür entwickelten Liposomen, wie sie von R.M. Hoffman in Journal of Drug Targeting, Vol. 5 (1997), 67 beschrieben sind. Hoffman beschreibt die Haarfollikel-selektive Einbringung von Makromolekülen bishin zum aktiven Gen mit einem Zielsystem auf Basis von topisch angewendeten Liposomen auf Phosphatylcholinbasis. Das System ist anwendbar auf in Liposomen eingebrachte oder daran gebundene Melanine, Proteine, Gene und dergleichen.

Kationische Lipide für die intrazelluläre Abgabe von biologisch aktiven Molekülen sind ferner aus WO-A 91/16024 bekannt, wobei diese kationischen Lipide für die topische, enterale wie auch parenterale Verabreichung von Wirkstoffen verwandt werden können.

In vielen Fällen kann es sinnvoll sein, den Wirkstoff systemisch zur Verfügung zu stellen, wie beispielsweise aus der Interferon-Therapie der multiplen Sklerose oder auch zur Behandlung von Diabetes bekannt. Diese Therapien beruhen auf der gleichmäßigen Verteilung des Wirkstoffs im Körper und der dadurch ausgelösten Wirkung. Der Wirkstoff kann dabei als solcher oder auch modifiziert eingesetzt werden, d.h. in Formen, die den eigentlichen Wirkstoff erst am Zielort freisetzen. Suspensionen für die parenterale Verabreichung enthalten den Wirkstoff zweckmäßigerweise ebenfalls gebunden an- oder eingeschlossen in Liposome als Transfermedium und suspendiert in einer physiologisch annehmbaren Trägerflüssigkeit.

Eine weitere systemische Darreichungsform ist beispielsweise in Form eines Nasalsprays, das den Wirkstoff auf die Schleimhäute bringt und über diese an den Körper abgibt.

Eine weitere Verabreichungsmögtichkeit ist die Implantation von pro- oder eukaryotischen Organismen oder den Wirkstoff enthaltenen Trägermaterialien, welche den Wirkstoff kontinuierlich oder reguliert sezemieren.

Schließlich sei auf die Möglichkeiten der Gentherapie verwiesen, d.h. die Einlagerung des Gens für DCoH/PCD in eine Zielzelle mittels DNA- oder RNA-Viren, die sich in das Genom integrieren und damit das Gen stabil einführen. Das Gen unterliegt dann der normalen Transkription und Translation in der Zelle und stellt damit eine sinnvolle Alternative zur transienten Transfektion mit dem Protein oder der dafür codierenden DNA oder RNA dar. Die Viren können sich dabei lokal einlagern - siehe auch die p53-Defizienz von Melanomen - oder sich ungerichtet im Körper verbreiten. Im Stand der Technik sind hierzu modifizierte Adenoviren oder andere "Erkältungsviren" beschrieben, die als geeignete Vehikel in Frage kommen. Zur Ausschaltung einer Hyperpigmentierung können Antisense-Techniken eingesetzt werden.

Es versteht sich, daß das Protein bzw. die dafür codierende DNA und/oder RNA zur Lagerung und Vermeidung von vorzeitiger Wirkungsminderung stabilisiert werden kann. Die Stabilisierung kann durch Beimischung üblicher Zusätze erreicht werden, so z. B. Puffersubstanzen, Salzen anderer Proteine wie auch von DNA und RNA erreicht werden. Aus der molekularen Biochemie sind hierzu beispielsweise Albumin, Heringssperma, DNA, tRNA bekannt, wie auch Detergenzien (ThesitR, TritonR), Alkali- und Erdalkaliionen und dergleichen. Eine Lagerung des Mittels und/oder Wirkstoffs in getrockneter oder gefriergetrockneter Form oder nach Schockfrieren im flüssigem Stickstoff kann ebenfalls sinnvoll sein.

Das erfindungsgemäße Mittel kann den Wirkstoff selbstverständlich in modifizierter Form enthalten, d.h. als Protein, in dem einzelne Aminosäuren ausgetauscht sind oder fehlen. Ebenso können in der DNA und/oder RNA Nukleotide oder Nukleotidsequenzen fehlen, ergänzt oder ausgetauscht werden. Voraussetzung ist, daß der so modifizierte Wirkstoff die von ihm verlangte Wirkung bei der Behandlung von Depigmentierungserscheinungen nach wie vor liefert. Gründe für solche Modifikationen können die Stabilisierung des Wirkstoffs, produktionstechnische oder formulierungstechnische Gründe oder auch eine Verbesserung der Wirkung oder des Wirkungsspektrums sein. In Frage kommen Mutation und Fusion nach gentechnischen oder chemischen Methoden, Fusionierungen mit N- und C-terminalen Proteinen oder Peptiden. Aus Gründen einer besseren Reinigung mittels Affinitätschromatographie können beispielsweise Histidintags oder Gst-Fusionen vorgenommen werden, oder Sequenzen eingefügt werden, um eine Dirigierung in bestimmte Zielzellen zu erleichtern - NLS-Sequenzen sind geeignet, den Wirkstoff in den Zellkern zu dirigieren. Durch Phosphorylierung oder Glykosilierung an geeigneten Resten, Modifikationen an der Phosphatgruppe der DNA oder RNA können Modifikationen vorgenommen werden, die dem Abbau durch körpereigene Nukleasen vorbeugen. Bekannt sind hier Phosphorthioatgruppen.

Zur Stabilisierung des Proteins kann es sinnvoll sein, Änderungen vorzunehmen, etwa bereits auf der Ebene der DNA/RNA, um beispielsweise Restriktionsstellen, chemische Instabilität oder Angriffspunkte von Nukleasen zu beseitigen oder die Wirkung zu verstärken oder zu spezifizieren, beispielsweise auch, um zwischen den verschiedenen Aktivitäten des multifunktionellen Proteins zu diskriminieren und eine störende Funktion stillzulegen. Umgekehrt kann es auch sinnvoll sein, die Stabilität zu vermindern und damit die Halbwertszeit des Wirkstoffs zu verkürzen, indem Modifikationen eingeführt werden, die den protolytischen Abbau ermöglichen, etwa durch Einbau von Erkennungssequenzen für Proteasen.

Das Protein kann auf übliche Weise nach Klonierung des Gens in geeignete Vektoren rekombinant aus Bakterien oder Eukaryonten isoliert werden. Die DNA und RNA kann aus Bakterien oder Eukaryonten nach Standardmethoden erhalten werden.

Das erfindungsgemäße Mittel zur Behandlung von Pigmentationsstörungen enthält den Wirkstoff in einem pharmazeutisch annehmbaren Trägermaterial, das einerseits aus mehreren üblichen Bestandteilen bestehen kann. Zweckmäßigerweise enthält das Trägermaterial wiederum ein für den Wirkstoff geeignetes Transfermedium, insbesondere ein Liposom oder Virus. Das Mittel ist in erster Linie für die topische oder parenterale Verabreichung bestimmt.

Die Erfindung betrifft neben einem Mittel zur Behandlung von Pigmentierungsstörungen auch die Verwendung von DCoH, der dafür codierenden DNA und/oder RNA oder von DcoH-Antikörpem bzw. -Antiserum als Wirkstoff zur Herstellung eines Medikaments zur Behandlung von Pigmentierungsstörungen des menschlichen und tierischen Körpers. Insbesondere werden unter Pigmentierungsstörungen Depigmentierungserscheinungen solche der Haut, Haare und der Retina verstanden, darunter insbesondere Vitiligo, Alopecia areata, sowie das vorzeitige oder altersbedingte Ergrauen des Haarwuchses und Formen von lokaler Überpigmentierung, etwa Melanodermie, Naevus und dergleichen. Die Erfindung betrifft sowohl angeborene wie erworbene Pigmentierungsstörungen.

Ein weiteres Anwendungsgebiet der erfindungsgemäßen DCoH, dafür kodierende DNA und/oder RNA oder DCoH-Antikörper oder -Antiserum enthaltenden Mittel liegt in der Behandlung von melanozytären Tumoren.

Melanozytäre Tumore und insbesondere auch das maligne Melanom stellen dedifferenzierte Melanozyten aus klonalem Ursprung dar.

Die Wirkung der erfindungsgemäßen Mittel auf melanozytäre Tumore geht in zweierlei Richtung. Zum einen beruht sie auf der Redifferenzierung von dedifferenzierten Melanozyten. Versuche an Xenopusoocyten haben gezeigt, daß DCoH die Differenzierung von Melanozyten einleitet. Die Anwendung der erfindungsgemäßen Mittel wirkt somit in Richtung auf eine Redifferenzierung und Normalisierung entarteter Zellen.

Zum anderen kann über Antikörper, Antiserum oder Antisense-RNA, gegebenenfalls auch über geeignete Mutanten der DCoH-DNA, -RNA oder des Proteins selbst, Einfluß auf die Teilung der Melanozyten genommen werden. Über die Blockierung der Wirkung des DCoH kann Einfluß auf die Teilungsgeschwindigkeit der Melanozyten bis hin zum Stillstand der Teilung und gegebenenfalls sogar zum Absterben der Zellen genommen werden.

Die Erfindung wird durch die nachfolgenden Beispiele näher erläutert, wobei hinsichtlich der Materialien und Methoden ergänzend auf E. Pogge von Strandmann und G.U. Ryffel, Development 121 (1995), 1217 bis 1226 hingewiesen wird.

### Beispiel 1:

Frösche der Gattung Xenopus wurden unter Standardbedingungen gehalten. Zur in vitro Befruchtung wurden 600 U Chorion Gonadotropin (Human) subkutan in den dorsalen Lymphsack geschlechtsreifer Weibchen injiziert und die Eier nach der Eiablage in Petrischalen mit intakten Spermien befruchtet. Nach 5 Minuten wurde mit 88 mM NaCl, 1 mM KCI, 0,7 mM CaCl₂, 1 mM MgSO₄, 5 mM Hepes pH 7,8 und 2,5 mM NaHCO₃ überschichtet.

Für die Mikroinjektion wurde die Gallerthülle entfernt und dann 1,5 Minuten mit 2 % Cystein bei pH 8,0 behandelt, anschließend in Überschichtungspuffer dreifach gewaschen. Nach der Injektion wurde bei Raumtemperatur in Überschichtungspuffer kultiviert.

Für die Injektion wurde durch in vitro Transkription aus geeigneten rekombinanten Vektoren gewonnene mRNA verwandt. Pro Ei wurden 500 pg/RNA in 25 nl Pufferlösung injiziert. Die dazu verwandten Glaskanülen wurden vor der Verwendung aus Kapillaren gezogen und hatten an der Spitze einen Durchmesser von nicht mehr als 30 µm.

Die sich aus den Eiern entwickelten Embryos zeigten eine deutliche Pigmentierung, die sich früher einstellte, als bei der Kontrolle. Die Pigmentierung trat zudem an Stellen auf, an denen der Embryo normalerweise nicht pigmentiert ist.

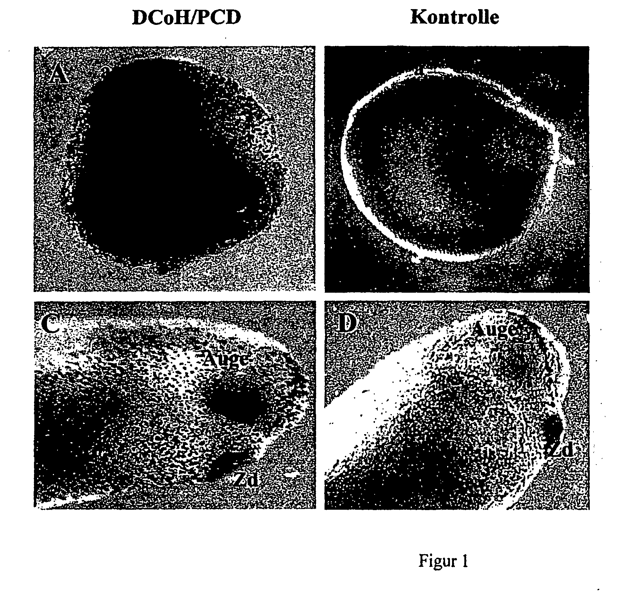

Fig. 1 zeigt Embryonen, die in der linken Bildreihe (A/C) DCoH überexprimieren. Auf der rechten Bildreihe (B/D) befindet sich die Kontrolle. Die dunklen Pigmentflecken sind in der linken Bildreihe deutlich zu erkennen. Es bezeichnen a = anima, eye = Auge, cg = cement glant.

DCoH/PCD induziert also ektopische Pigmentzellen. Das Ergebnis zeigt, daß DCoH/PCD allein, d.h. ohne die Melaninsynthesekette, in der Lage ist, die für die Pigmentsynthese notwendigen Schritte auszulösen.

### Beispiel 2:

In diesem Beispiel wird die RNA für DCoH in Xenopus-Embryonen im 2-Zellstadium in nur eine der beiden Zellen injiziert, zusammen mit der RNA für GFP, green fluorescent protein. Die Coinjektion der RNA für GFP diente der Visualisierung, daß die injizierten RNAs auch nur in den injizierten Zellen in aktives Protein translatiert werden.

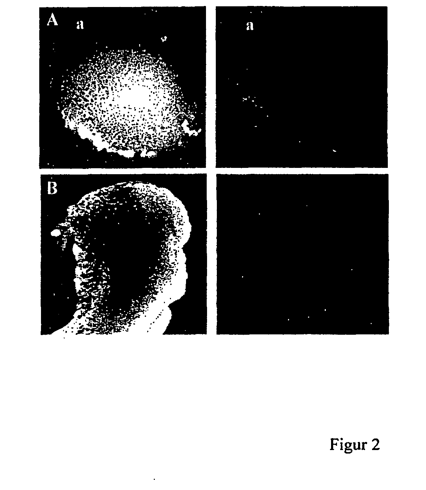

In der sich aus dem zweiten Zellstadium entwickelnden Larven läßt Fig. 2 erkennen, daß die Pigmentierung auf eine Körperhälfte der Larve beschränkt ist, dort, wo auch die Fluoreszenz (rechte Bildreihe) sichtbar ist. DCoH/PCD ist somit geeignet, in den damit beaufschlagten Zellen eine vorzeitige und über das normale Maß hinausgehende Pigmentierung auszulösen. Die Larven entwickelten sich, abgesehen von der Hyperpigmentierung und der Fluoreszenz normal.

### Beispiel 3:

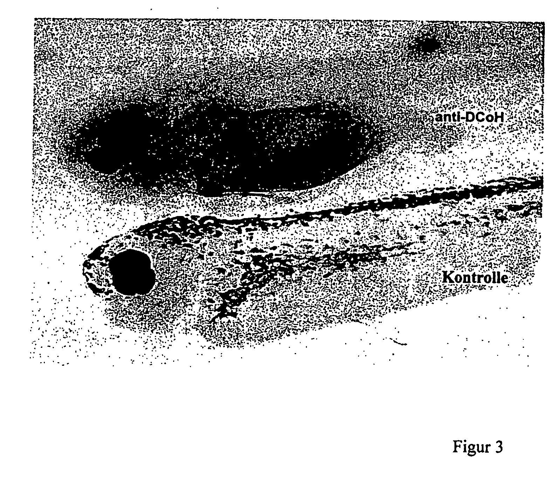

Nach Standardmethoden im Kaninchen gewonnenes Antiserum gegen DCoH wurde analog zu Beispiel 2 in Zellen von Fröschen der Gattung Xenopus injiziert. Fig. 3 zeigt, daß in drei Tage alten Embryonen die Pigmentbildung völlig unterdrückt ist (α-DcoH, oberes Bild), während in der Kontrolle (unteres Bild) die normale Pigmentierung auftritt.

## Patentansprüche

1. Mittel zur Behandlung von Pigmentierungsstörungen des menschlichen und tierischen Körpers,
**dadurch gekennzeichnet, daß** es eine physiologisch wirksame Menge des Proteins DCoH, (Dimerisierungscofactor des "Hepatocyte Nuclear Factor-1 (HNF-1)) der dafür codierenden DNA und/oder RNA oder von DcoH-Antikörpem oder -Antiserum, in einem pharmazeutisch annehmbaren Trägermaterial enthält.

2. Mittel nach Anspruch 1, **dadurch gekennzeichnet, daß** es das Protein DCoH enthält.

3. Mittel nach Anspruch 1, **dadurch gekennzeichnet, daß** es DcoH-Antikörper enthält.

4. Mittel nach Anspruch 1, 2 oder 3, **dadurch gekennzeichnet, daß** das Trägermaterial ein den Wirkstoff transportierendes Transfermedium enthält.

5. Mittel nach Anspruch 4, **dadurch gekennzeichnet, daß** das Transfermedium eine Liposomzubereitung oder ein Virus ist.

6. Mittel nach einem der vorstehenden Anspräche, **dadurch gekennzeichnet, daß** es als Formulierung zur topischen Anwendung vorliegt.

7. Mittel nach einem der Ansprüche 1 bis 4, **dadurch gekennzeichnet, daß** es als Injektionsformulierung vorliegt.

8. Mittel nach einem der vorstehenden Ansprüche, **dadurch gekennzeichnet, daß** es zur Pigmentierung oder Depigmentierung von Haut oder Haaren bestimmt ist.

9. Mittel nach Anspruch 8, bestimmt zur Behandlung von Vitiligo, Alopecia areata, und/oder Canities bzw. Melanodermie.

10. Verwendung des Proteins DCoH, der dafür codierenden DNA und/oder RNA, oder von DcoH-Antikörpern bzw. -Antiserum, als Wirkstoff zur Herstellung eines Mittels nach einem der Ansprüche 1 bis 8 zur Behandlung von Pigmentierungsstörungen des menschlichen und tierischen Körpers.

## Claims

1. A preparation for the treatment of pigmentation disorders of the human and animal body, **characterized in that** it contains a physiologically active amount of the protein DCoH (the dimerisation co-factor of the "Hepatocyte Nuclear Factor-1 (HNF-1)), the DNA and/or RNA coding for said protein, or DCoH antibodies or DCoH antiserum in a pharmaceutically acceptable excipient.

2. The preparation according to claim 1, **characterized in that** it contains the protein DCoH.

3. The preparation according to claim 1, **characterized in that** it contains DCoH antibodies.

4. The preparation according to claims 1, 2 or 3, **characterized in that** the excipient contains a transfer medium transporting the active ingredient.

5. The preparation according to claim 4, **characterized in that** the transfer medium is a liposome preparation or a virus.

6. The preparation according to any one of the preceding claims, **characterized in that** it is present in the form of a formula for topical application.

7. The preparation according to any one of claims 1 to 4, **characterized in that** it is present in the form of an injection formula.

8. The preparation according to any one of the preceding claims, **characterized in that** it is intended for the pigmentation or de-pigmentation of skin or hair.

9. The preparation according to claim 8 intended for the treatment of vitiligo, alopecia areata and/or canities or melanoderma.

10. The use of the protein DCoH, the DNA and/or RNA for the coding of said protein, or DCoH antibodies or DCoH antiserum, as active ingredient for producing a preparation according to any one of claims 1 to 8 for the treatment of pigmentation disorders of the human or animal body.

## Revendications

1. Produit pour le traitement d'altérations de la pigmentation du corps humain et animal, **caractérisé en ce qu'**il contient une quantité physiologiquement efficace de protéines DCoH (cofacteur de dimérisation de l' "Hepatocyte Nuclear Factor-1" (HNF-1)), d'ADN et/ou d'ARN codant pour celles-ci ou d'anticorps ou d'antisérum de DcoH, dans un matériau support pharmaceutiquement acceptable.

2. Produit selon la revendication 1, **caractérisé en ce qu'**il contient la protéine DCoH.

3. Produit selon la revendication 1, **caractérisé en ce qu'**il contient des anticorps de DcoH.

4. Produit selon la revendication 1,2 ou 3, **caractérisé en ce que** le matériau support contient un milieu de transfert véhiculant la substance active.

5. Produit selon la revendication 4, **caractérisé en ce que** le milieu de transfert est une préparation de liposome ou un virus.

6. Produit selon l'une des revendications précédentes, **caractérisé en ce qu'**il se présente en formulation pour utilisation topique.

7. produit selon l'une des revendications 1 à 4, **caractérisé en ce qu'**il se présente en formulation pour injection.

8. Produit selon l'une des revendications précédentes, **caractérisé en ce qu'**il est destiné à la pigmentation ou la dépigmentation de la peau ou des cheveux.

9. Produit selon la revendication 8, destiné au traitement du vitiligo, de la pelade, et/ou de canitie ou de mélanodermie.

10. Utilisation de la protéine DCoH, d'ADN et/ou d'ARN codant pour celle-ci ou d'anticorps ou d'antisérum de DcoH, comme substance active pour la préparation d'un produit selon l'une des revendications 1 à 8 pour le traitement d'altérations de la pigmentation du corps humain et animal.
